# EUROPÄISCHE PATENTSCHRIFT

(11) **EP 0 865 794 B1**
(45) Veröffentlichungstag und Bekanntmachung des Hinweises auf die Patenterteilung: **16.07.2003**
(21) Anmeldenummer: 98890075.9
(22) Anmeldetag: 19.03.1998
(51) Int. Cl.: A61M 1/28

(54) **Vorrichtung zum Entsorgen von Flüssigkeiten im medizinischen Bereich**
Medical liquid disposal device
Dispositif médical d'évacuation de liquides

(30) Priorität: 20.03.1997 AT 17097
(43) Veröffentlichungstag der Anmeldung: 23.09.1998
(73) Patentinhaber: Hageneder, Konrad, 4643 Pettenbach (AT)
(72) Erfinder: Hageneder, Konrad, 4643 Pettenbach (AT)
(74) Vertreter: Hübscher, Gerhard, Dipl.-Ing.

(56) Entgegenhaltungen:
- EP-A- 0 112 104
- WO-A-95/20985
- US-A- 4 412 917
- US-A- 4 560 472

## Beschreibung

Die Erfindung bezieht sich auf eine Vorrichtung zum Entsorgen von Flüssigkeiten im medizinischen Bereich, insbesondere von Dialysaten, mit einem, eine Ein- und Auslauföffnung aufweisenden Flüssigkeitsbehälter, wobei als Behälter ein selbsttragender, auf Stützrädern fahrbar abgestützer Kanister vorgesehen ist. Bekannte Einrichtungen bestehen darin, daß das verbrauchte Dialysat in ca. 15 Liter fassenden Kunststoffsäcken aufgefangen wird, die nach der Behandlung zu einer Entsorgungsstelle (WC, Badewanne,...) gebracht, dort entleert, und anschließend entsorgt werden müssen. Weiters ist uns das EP 112104A2 eine Einrichtung zur Peritonealdialyse bekannt geworden, die das Auffangen und zur Entsorgungsstelle Transportieren des Dialysates mit einer mit kleinen Rädern ausgestatteten Wanne durchführt.
Der Erfindung liegt die Aufgabe zu Grunde, das Entsorgen von Dialysaten zu erleichtern, insbesondere das Tragen der 15 Kilo schweren Kunststoffsäcke und die Verwendung von Einmalmaterialien (insbesondere PVC) zu vermeiden.

Die Erfindung löst diese Aufgabe dadurch, daß an der im Bodenbereich angeordneten Auslauföffnung (2) des Kanisters (1) ein Auslaufschlauch (3) angesetzt ist, der in einem Auslaufrohr (4) endet, und daß zusätzlich zur im oberen Kanisterbereich angeordneten Einlauföffnung (11) eine Druckluftanschlußstelle (8) vorgesehen ist, an der ein bedarfsweise druckluftbeaufschlagbarer Druckschlauch anschließbar ist. Das Befüllen des Kanisters erfolgt über die Einlauföffnung (11), welche anschließend mit einer Verschlußkappe verschlossen wird. Der Kanister kann auf Grund der vorhandenen Stützräder (9) und des Griffes (10) leicht zu einer Entsorgungsstelle gefahren werden. Zum Entleeren des Kanisters muß die Verschlußkappe (7), die mit einer Kette oder dergleichen am Kanister befestigt ist (Anspruch 5), am Auslaufrohr entfernt, und das Auslaufrohr, das vertikal schwenkbar mit Klemmen am Kanister befestigt ist (Anspruch 3), über einer Entsorgungsstelle (WC, Badewanne,...) in Auslaufstellung gebracht werden (13).
Durch das in Auslaufstellung gebrachte Auslaufrohr wird die Druckluftanschlußstelle (8), die sich hinter dem Horizontalabschnitt des Auslaufrohres (5) befindet, zugänglich (Anspruch 4), und es kann ein Druckluftschlauch an das Rückschlagventil der Druckluftanschlußstelle (8) angeschlossen werden.
Durch Anspruch 4 und 5 wird sichergestellt, daß, solange das Auslaufrohr mit einer Verschlußkappe verschlossen ist, kein Druckluftschlauch an der Druckluftanschlußstelle angeschlossen werden kann, weil die Kette oder dergleichen, mit der die Verschlußkappe am Behälter befestigt ist, ein Wegschwenken (in Auslaufstellung bringen) des Auslaufrohres verhindert. Wenn der Druckluftschlauch angeschlossen ist, wird mit einem Kompressor Luft in den oberen Teil des Kanisters eingebracht. Durch den Überdruck im oberen Teil des Kanisters, wird die sich darin befindende Flüssigkeit über die Auslauföffnung (2) im unteren Teil des Kanisters, aus dem Kanister gepreßt. Nach der vollständigen Entleerung wird der Druckluftschlauch entfernt, dasAuslaufrohr am Kanister wieder angeschwenkt (Außerbetriebstellung), und mit der Verschlußkappe (7) wieder verschlossen.

Der Kanister ist zur mehrmaligen Verwendung bestimmt, und muß je nach Verwendungsbereich entsprechend gereinigt oder eventuell desinfiziert werden.

Ein Ausführungsbeispiel der Erfindung ist in der Zeichnung dargestellt, Fig. 1 eine erfindungsgemäße Vorrichtung in Vorderansicht, Fig. 2 diese Vorrichtung in Seitenansicht, Fig. 3 in Draufsicht.

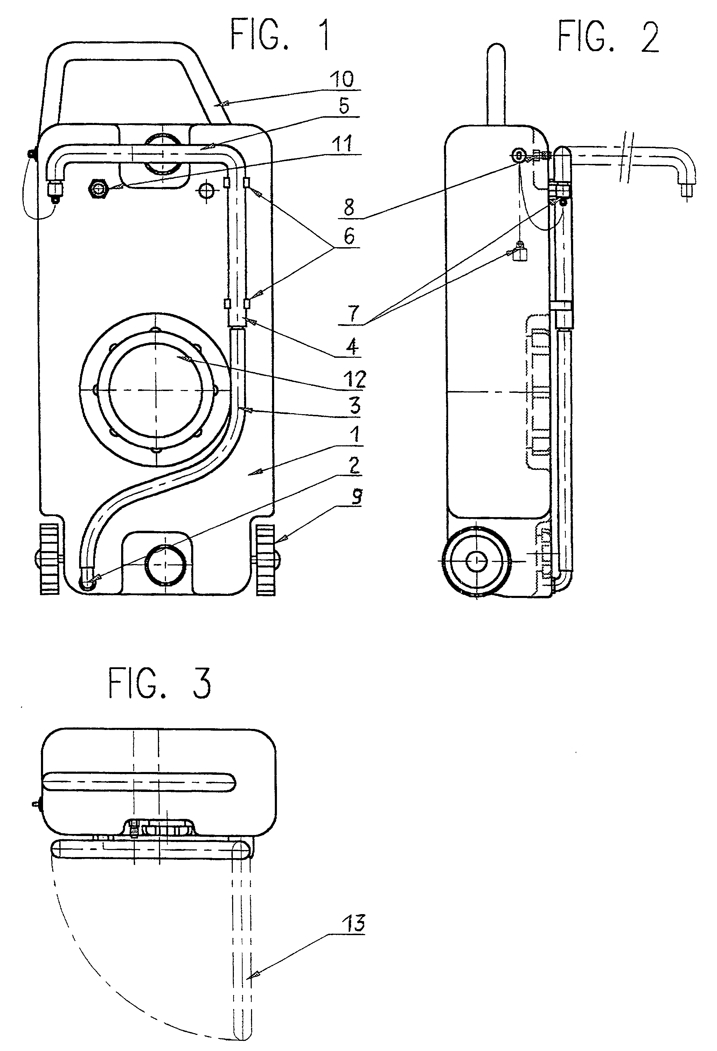

Die Vorrichtung besteht aus einem Behälter (1), der als selbsttragender, auf Stützrädern (9) fahrbar abgestützter Kanister ausgeführt ist. Fig. 1 zeigt die Auslauföffnung (2) im Bodenbereich des Kanisters, an die ein Auslaufschlauch (3) ansetzt, der in ein Auslaufrohr (4) übergeht. Dieses Auslaufrohr (4) ist mit zwei Klemmen (6) am Kanister befestigt, sodaß man es vertikal schwenken kann (Position 13). Das Auslaufrohr (4) geht in einen Horizontalabschnitt (5) über, dahinter befindet sich die Druckluftanschlußstelle (8). Am Auslaufrohrende befindet sich eine Verschlußkappe (7), die mit einer Kette oder dergleichen am Kanister derart befestigt ist, daß das Auslaufrohr (4) im verschlossenen Zustand nicht vom Kanister weggeschwenkt werden kann, und die Druckluftanschlußstelle (8) nicht zugänglich ist.
Weiters ist die Einlauföffnung (11) dargestellt, durch die Flüssigkeit in den Kanister eingefüllt wird, die Einlauföffnung (11) ist mit einer Verschlußkappe versehen.
Weiters ist eine große Reinigungsöffnung (12) dargestellt, die eine optimale Reinigung des Kanisters gewährleistet.
In Fig. 1 ist der Griff (10) dargestellt, mit dem der Kanister gehalten werden kann.

## Patentansprüche

1. Vorrichtung zum Entsorgen von Flüssigkeiten im medizinischen Bereich, insbesondere zum Entsorgen von Dialysaten, mit einem, eine Ein- und Auslauföffnung (11, 2) aufweisenden Flüssigkeitsbehälter, wobei als Behälter ein selbsttragender, auf Stützrädern (9) fahrbar abgestützter Kanister (1) vorgesehen ist, **dadurch gekennzeichnet, daß** an dessen im Bodenbereich angeordneten Auslauföffnung (2) ein Auslaufschlauch (3) angesetzt ist, der in einem Auslaufrohr (4) endet, und daß zusätzlich zur im oberen Kanisterbereich angeordneten Einlauföffnung (11) eine Druckluftanschlußstelle (8) vorgesehen ist, an der ein bedarfsweise dmckluftbeaufscblagbarer Druckschlauch anschließbar ist.

2. Vorrichtung nach Anspruch 1 **dadurch gekennzeichnet, daß** die Druckluftanschlußstelle (8) mit einem Rückschlagventil versehen ist.

3. Vorrichtung nach Anspruch 1 oder 2 **dadurch gekennzeichnet, daß** das Auslaufrohr um eine Vertikalachse (6) schwenkbar am Kanister (1) anlenkbar ist.

4. Vorrichtung nach Anspruch 3, **dadurch gekennzeichnet, daß** das Auslaufrohr (4) einen in Höhe der Druckluftanschlußstelle (8) liegenden Horizontalabschnitt (5) bildet, der von einer abstehenden Auslaufstellung (13) in eine die Druckluftanschlußstelle (8) abdeckende Außerbetriebsstellung an den Kanister (1) anschwenkbar ist.

5. Vorrichtung nach einem der Ansprüche 1 bis 4, **dadurch gekennzeichnet, daß** das Auslaufrohr (4) mit einer Kappe (7) verschließbar ist, die mit einer Kette oder dergleichen am Kanister (1) angehängt ist.

## Claims

1. A device for disposing of liquids in the medical sector, more particularly for disposal of dialysates comprising a liquid container having an inlet opening and an outlet opening, the container being a self-supporting wheeled canister, **characterised in that** a hose (3) is mounted on the outlet opening (2), which is disposed near the ground, and ends in a pipe (4) and in addition to the inlet opening (11), which is disposed in the upper region of the canister, a compressed air connection (8) is provided for connecting to a pressure hose which can be supplied with compressed air when required.

2. A device according to claim 1, **characterised in that** the compressed-air connection (8) has a non-return valve.

3. A device according to claim 1 or 2, **characterised in that** the outlet pipe is mounted on the canister (1) so as to be pivotable around a vertical axis (6).

4. A device according to claim 3, **characterised in that** the outlet pipe (4) has a horizontal portion (5) level with the compressed-air connection (8) and pivotable on the canister (1) from a projecting outlet position (13) into an inoperative position ccvering the compressed-air connection (8).

5. A device according to any of claims 1 to 4, **characterised in that** the outlet pipe (4) is closable by a cap (7) suspended by a chain or the like on the canister (1).

## Revendications

1. Dispositif d'élimination de liquides dans le domaine médical, en particulier pour éliminer des dialysats, avec un récipient à liquides présentant une ouverture d'introduction et d'évacuation (11, 2), étant prévu comme récipient un bidon (1) autoporteur, soutenu de façon déplaçable sur des roues d'appui (9), **caractérisé par le fait qu'**un tuyau d'évacuation (3) s'achevant en un tuyau de sortie (4) est monté sur l'ouverture de sortie (2) disposée dans la zone de fond et **par le fait que**, en plus de l'ouverture d'entrée (11) disposée dans la zone supérieure du bidon est prévu un point de raccordement d'air comprimé (8), auquel en cas de besoin un tuyau à pression alimenté en air comprimé peut être raccordé.

2. Dispositif selon la revendication 1, **caractérisé par le fait que** le point de raccordement d'air comprimé (8) est muni d'un clapet anti-retour.

3. Dispositif selon la revendication 1 ou 2, **caractérisé par le fait que** le tuyau d'évacuation est susceptible d'être articulé sur le bidon (1), de façon pivotante autour d'un axe vertical (6).

4. Dispositif selon la revendication 3, **caractérisé par le fait que** le tuyau de sortie (4) forme un tronçon horizontal (5), situé au niveau du point de raccordement en air comprimé (8), tronçon susceptible d'être approché, par pivotement, d'une position d'évacuation (13), en saillie, en une position hors service, couvrant le point de raccordement en air comprimé (8), sur le bidon (1).

5. Dispositif selon l'une des revendications 1 à 4, **caractérisé par le fait que** le tube d'évacuation (4) est susceptible d'être obturé avec un capuchon (7), accroché au bidon (1) à l'aide d'une chaîne ou analogue.
